# EUROPEAN PATENT APPLICATION

(11) **EP 4 173 671 A1**
(43) Date of publication of application: **03.05.2023**
(21) Application number: 21205787.1
(22) Date of filing: 01.11.2021
(51) Int. Cl.: A61N 1/372, G16H 40/40

(54) **PATIENT TRIGGERED PROGRAM SELECTION FOR IMPLANTS**

(71) Applicant: BIOTRONIK SE & Co. KG, 12359 Berlin (DE)
(72) Inventor: MÜLLER, Jens, 14195 Berlin (DE); DEUTSCHMANN, Hendrik, 12435 Berlin (DE); DÖRR, Thomas, 12437 Berlin (DE); SELENT, Miro, 14558 Nuthetal (DE)

(57) **Abstract**

The present disclosure relates to apparatuses and methods for adjusting settings of an implantable cardiac rhythm management, CRM, device. A patient device may comprise a memory with a list of available programs of the implantable CRM device and a user interface for selecting, by the patient, one or more of the available programs. It may further comprise a transmitter for sending a query to the implantable CRM device to switch to the one or more selected programs.

## Description

The present disclosure relates to apparatuses and methods for adjusting settings of an implantable cardiac rhythm management, CRM, device and interaction possibilities of a patient with the implanted CRM device.

Heart issues of a patient (such as e.g. cardiac arrhythmia, sick sinus syndrome, Tachycardia, etc.) may be drastically reduced or even completely compensated by implantable CRM devices. Such implants are typically inserted/implanted into the patient's body during a surgery and/or by means of a catheter-based implantation, e.g., through one of the principal arteries.

The implants typically possess a programming which, when executed, causes the implant to fulfil its envisaged task (e.g. ensuring a regular heartbeat pattern, if the implant, e.g., relates to a pacemaker). The programming typically relates to a pre-defined programming, e.g., by the manufacturer or by the surgeon at the implanting process.

However, this "common" procedure comprises several drawbacks. The rather "standardized" programming of the implanted medical devices not always captures the individual needs and requirements of the patient and therefore does not always achieve the optimum result. A later programming or fine tuning of the implanted medical device is not always foreseen or at most only during seldom check-ups with limited influence by the patient. For example, US 2020/0139141A1 relates to implantable medical devices that may be in communication with clinician programmer devices.

However, a CRM implant confronts a patient with a radically new situational context in life. The level of acceptance and trust with which the patient perceives the implant is oftentimes rather low or at least agitates the patient as there is typically no possibility to interact with the implant. This is in particular of relevance for implantable CRM devices, such as, e.g., pacemakers, defibrillators, etc., as a misunderstanding or lack of trust with these devices may immediately worry the patient as life-threatening event.

Based on the drawbacks of current CRM devices as outlined above and the absence of solutions for these drawbacks in the prior art, a need for improvements of CRM devices arises.

According to an aspect of the present disclosure, the above need is at least partly met by a patient device for adjusting settings of an implantable cardiac rhythm management, CRM, device. The patient device may comprise a memory with a list of available programs of the implantable CRM device, and it may comprise a user interface for selecting, by the patient, one or more of the available programs. The patient device may further comprise a transmitter for sending a query to the implantable CRM device to switch to the one or more selected programs.

Hence, the CRM device is not limited to a single specific programming as in current CRM devices which do not allow the patient to influence or interact with the implant. Instead, the patient is enabled to change the operation mode of the CRM device (e.g. within bounds foreseen by the programmer) by switching to a suitable program. The patient device may in particular allow an adjustment of the CRM device by the patient, e.g., at home in the absence of any medical staff. This may first of all provide the advantage that the patient may be at home where the overall comfort for the patient is maximized as no hospitalization is necessarily required for the adjustment of the CRM device. Second, this provides the opportunity to adjust the implant according to the individual and in particular temporal needs of the patient, e.g., according to a mood and/or a planned activity, such as a sporting activity, lying, climbing stairs, a certain mood, etc., which may each benefit from (slightly) different settings for the CRM device. Since the patient may be allowed to adjust the CRM device according to the individual needs, the patient may also experience how the CRM device reacts to certain adjustments which may therefore provide a learning effect for the patient and may thus allow accustoming of the patient to the implanted device. This may also increase the level by how much the patient trusts the CRM device (e.g. overcoming feelings of insecurity) as well as the level of acceptance of the device (e.g. causing the patient to identify with the CRM device). Moreover, since the patient is not exclusively bound to the specific programming of the CRM device as obtained during the implantation procedure (or a check-up), a more patient-specific and individual effect of the CRM device on the health state of the patient may be achieved. In other words, by allowing the patient to adjust the CRM device, the therapy to be performed by the CRM device may be closely tailored to the individual health state and environmental conditions of the patient.

A patient device in the context of the present application may be understood as a device which is suitable for being carried by the patient (and/or a relative and/or a caretaker of the patient). Such a device may e.g. be a mobile phone (e.g. a smartphone which may execute a dedicated app), a tablet, a dedicated handheld device (i.e. a device which has exclusively been developed to interact with the implantable CRM device), a wearable (e.g. a smartwatch or any other bracelet-like device which is capable of providing the required functionality as mentioned above), a smart home device, and/or any suitable mobile and/or handheld device.

An implantable CRM device in the context of the present application may be understood as any implant which is capable of monitoring and/or affecting (active CRM device) cardiac parameters of the patient. An implantable CRM device may thus relate to a pacemaker, a defibrillator, for example an implanted cardioverter-defibrillator (ICD), e.g. a cardiac resynchronization therapy defibrillator (CRT-D) or pacemaker (CRT-P), etc.

According to an aspect, the patient device may further comprise a receiver for receiving a current list of available programs of the implantable CRM device from the implantable CRM device. Optionally, the transmitter of the patient device may further be configured to send a request to the implantable CRM device requesting the current list of available programs.

Hence, the patient device may be enabled to carry out the selection step based on the current list of programs, and/or update its list of available programs accordingly. This particularly allows updates of an implanted CRM device, since, by receiving the current list from the CRM device, the patient device may be enabled to always correctly select an actually available program, such that errors and miscommunication may be avoided.

The request may be understood as a command (e.g. a message) sent to the implantable CRM which prompts the CRM device to determine all available programs which may be selected by the patient device and/or retrieve a corresponding list already stored on the CRM device. The request may further comprise a command to return the list of available programs to the patient device.

The sending of the request may be performed upon initiating the request by the patient, e.g. by a corresponding selection on the patient device. It may additionally or alternatively be possible that the request is sent on a pre-defined basis (e.g. based on a pre-defined schedule such as, e.g., pre-defined dates). The pre-defined basis may also refer to a periodic sending of the request, such as once per hour, once per day, once per week, once per month, once per year or any other suitable periodic basis.

In other examples, a request may not be sent, but the current list may be received automatically, e.g., whenever the programs of the CRM device change, on a pre-defined basis, etc.

It is noted that the term "list" as used herein is not intended to require any specific data structure. It is only used to outline that the patient device is informed about all currently available programs of the CRM device which may be selected by the patient device, such that the patient, via the patient device's user interface, is enabled to select from these.

The available programs may be stored on the implantable CRM device. The available programs may relate to a fixed set of programs which does not change over time (e.g. OEM programs). It may also be possible that at least a portion of the available programs may be stored on the implantable CRM in the context of implantation and/or post-surgery, e.g. during a check-up. Therefore, the number of available programs and the programs themselves may change over time. By requesting and/or receiving the current list of available programs, it may be ensured that the patient device is constantly in an updated state, i.e. the patient device may be informed about any possible available program and/or program changes and/or additional programs.

Another aspect relates to an implantable cardiac rhythm management, CRM, device, which may comprise a memory with available programs of the implantable CRM device and which may comprise a receiver for receiving, from a patient device, a query to switch to one or more of the programs.

The CRM device may thus be programmed flexibly, having stored thereon, several programs. Without interaction with a programmer or a further programming step, the patient may hence change the operation mode of the CRM device, by simply sending a corresponding query to the CRM device. In other words, the CRM device's operation may flexibly be adjusted according to the individual patient's needs within the boundaries provided by the programming (e.g. within the boundaries as enabled by the various programs stored on the CRM device). The query from the patient device may comprise a command to switch from a current program of the implantable CRM to another program of the implantable CRM

The implantable CRM device may further comprise a transmitter for sending, to the patient device, a current list of available programs of the implantable CRM device. Optionally, the receiver of the implantable CRM device may further be configured for receiving a request, from the patient device, requesting the current list.

Sending the current list to the patient device may ensure that the patient device always selects a program that is actually currently available, such that communication errors may be excluded. This is particularly beneficial in cases in which the programs of the CRM device are altered or updated or if new programs are added while the CRM device is implanted.

Another aspect relates to a programming device for programming an implantable cardiac rhythm management, CRM, device, which may comprise a transmitter for sending programming data to the implantable CRM device. The programming device may additionally be adapted to provide the implantable CRM device with a set of programs.

Hence, the CRM device may be provided with flexibility. Instead of providing only a single program, a set of programs is supplied, such that the CRM device's operation mode may be altered by selection of a suitable program as needed, e.g. by a patient device in communication with the CRM device, while the CRM is implanted, and without the need for further programming.

The programming device may comprise a user interface that allows, e.g., a surgeon and/or a programmer to create and/or modify programs and/or subprograms. For example, one or more (sub-)programs provided from a manufacturer of the implant may be modified by the programming device and/or programs may be created completely anew (e.g. within bounds as specified by the manufacturer).

A program may be understood to include programming code with certain instructions. It is noted that a first program may differ from a second program by setting one or more operating parameters of the CRM device differently. Thus, selecting a program, by the patient, may for example change one or more operating parameters of the CRM device. In other examples, changing a program may lead to a more fundamental change in the operation mode of the CRM device. A first and a second program may for example be understood as first and second subprogram, i.e. whenever the present application refers to a program, also the term sub-program is understood as being comprised by that term.

It may be possible that a program fixes all operating parameters of the CRM device. However, it may also be possible that a program defines a range or selected values for one or more operating parameters which may then be fine-tuned by further selection of the patient. In other words, by selecting the respective program (on the patient device), the patient may be prompted to carry out a further selection for one or more operating parameters (from a range and/or predetermined values). The patient device may then send a query to the CRM device to switch to the selected program with the further selections by the patient.

It may also be possible that the implantable CRM device runs a main program. The main program may additionally be accompanied or replaced by a sub-program (the sub-program may be programmed by medical staff), if selected and optionally fine-tuned by e.g. the patient on e.g. the patient device. The sub-program may thus be understood as a program which may overwrite some settings of the main program (e.g. settings which are associated with the adjustment of a heart rate) such that e.g. one or more of the operation parameters of the implantable CRM device are replaced by the operation parameters defined in the sub-program.

When selecting a sub-program, the sub-program may overwrite some of the settings of the main program for an unlimited amount of time or for a predetermined (e.g. by the patient, the medical staff and/or an OEM value) period of time. After the expiration of the predetermined period of time, the implantable CRM device may automatically return to the main program accompanied by (re-) applying the respective settings defined therein. The trigger in response to which the implantable CRM device returns to the main program may additionally or alternatively be based on a respective request received from the patient device to return to the main program. Additionally or alternatively it may also be possible that the implantable CRM device returns to the main program if the implantable CRM device detects an event (e.g. a certain vital parameter (heart rate) of the patient surpasses or undercuts a pre-defined threshold).

Generally, after switching from a first program to a second program, the CRM device may fallback to the first program after a predetermined time has lapsed (e.g. as outlined above). Also, such fallback may occur in case the CRM device detects an event (e.g. as outlined above). Still, the second program may be run, e.g., until a further query to switch to yet another program is received by the CRM device.

The transmitter of the programming device may further be adapted to send the programming data to the implantable CRM device remotely, via a remote network service and/or a relay device. The relay device may be in direct communication with an implanted CRM device. Such relay device may be paired with the implanted CRM device, such that the CRM device may be enabled for direct communication with that relay device and/or to only communicate with a single relay device.

This allows updating and/or adding new programs without the patient having to visit a hospital. Rather, the CRM device may be provided with new, improved (potentially patient-specific) functionality "over the air", without affecting the patient. Nevertheless, since the present disclosure foresees providing the patient device with the current list of available programs, it is always ensured that the patient device may make proper selections out of the list of programs that are actually currently available.

The remote network service may e.g. relate to a hospital information system (e.g. accessible over the internet or in a local network), a network service supplied by the manufacturer of the implantable CRM device, a 3^{rd} party cloud service, or any other suitable remote network service.

The remote network service may provide a pool of potentially available programs for an implantable CRM device, and/or store the programs received from programming devices. In turn, the programming devices may retrieve programming data from such network service, wherein suitable programs for the patient may then be selected by the medical staff. Upon a selection, the respective program may be provided to the implantable CRM device as an available program.

In other examples, the programming data may be sent to the implantable CRM device directly, e.g., by means of near-field communication (NFC), W-LAN, Bluetooth or any other suitable communication method (e.g., without any further devices involved). This may for example be done during implantation or a check-up in a hospital.

A programming device, as described herein, may be implemented to provide a remote and/or a direct communication path with the implantable CRM device. For example, when sending programming data to the implantable (or implanted) CRM device directly, it may at the same time also be transmitted to the remote network service, such that the latter is also provided with the programming data. This may provide the benefit that the respective new, changed or updated program(s) may also be available for retrieval from the remote network service for other patients. Another benefit may be that the remote network service is simply always up-to-date concerning the current programs of the CRM device (such that it may provide a current list of programs to a patient device, without first having to inquire with the CRM device).

Another aspect relates to a programming device for programming an implantable cardiac rhythm management, CRM, device. The programming device may comprise a user interface to a remote network service. The user interface may be configured to cause the remote network service to provide the implantable CRM device with a set of programs.

This may allow the programming device to be a very simple device, e.g. a tablet, which only provides a user interface to the remote network service. Using this interface, programs can be imported into the remote network service from remote sources or from a source within the remote network service themselves, and then sent from there to the implantable CRM device. The programs may be generated or, if available, selected within the remote network service. A decentralized programming device in the clinic could thus be omitted.

It is noted that this aspect may be combined with any of the further aspects outlined herein with reference to a programming device.

Similarly, an implantable (or implanted) CRM device may be implemented such that it provides direct communication (e.g. NFC, W-LAN, Bluetooth) with the programming device and/or the patient device and/or a relay device. Additionally or alternatively, the CRM device may communicate with the programming device remotely, e.g. via the relay device which is in communication with a remote network service (e.g. via the internet). Still additionally or alternatively, the CRM device may communicate with the patient device indirectly and/or remotely, e.g. via the relay device which may be in communication with a remote network service (e.g. via the internet).

Also, a patient device may be implemented such that it provides direct communication (e.g. NFC, W-LAN, Bluetooth) with the implantable CRM device. Additionally or alternatively, the patient device may communicate with the implantable CRM device indirectly, e.g. via a relay device (e.g. NFC, W-LAN, Bluetooth), or remotely, e.g. via a remote network service (e.g. via the internet) and a relay device.

The CRM device and/or the patient device, just as the programming device, may thus also be provided with interfaces for direct, indirect and/or remote communication (with each other and/or with the programming device, respectively).

Another aspect of the present invention addresses a system for adjusting settings of a cardiac rhythm management, CRM, device. The system may comprise the patient device as outlined herein and may comprise at least one of the implantable cardiac rhythm management, CRM, device as outlined herein and/or the programming device for programming an implantable cardiac rhythm management, CRM, device as outlined above.

Another aspect relates to a system comprising a patient device and an implantable CRM device (and/or optionally a programming device) as described herein. When implementing such a system, an integrally implemented patient-adjustable CRM system may be supplied. This provides the advantage that all devices may preferably be developed and supplied by a single manufacturer which may increase the reliability of the system (e.g., increased life span, decreased maintenance efforts (check-up) and costs, etc.) and may provide increased support capability by a single manufacturer. Additionally, potential compatibility problems (e.g., suddenly aborting data transmission, etc.) may be reduced.

The implantable CRM device and the patient device may be paired for enabling direct communication between the implantable CRM device and the patient device, and/or to make sure that the CRM device can only be adjusted by that (single) patient device.

Another aspect relates to a method, at a patient device, for adjusting settings of an implantable cardiac rhythm management, CRM, device, comprising selecting, based on patient input provided via a user interface of the patient device, one or more programs from a list of available programs for the implantable CRM device, wherein the list is stored in a memory of the patient device. The method may further comprise sending a query to the implantable CRM device to switch to the one or more selected programs.

The method at the patient device may further comprise receiving a current list of available programs of the implantable CRM device from the implantable CRM device. Optionally, it may further comprise sending a request to the implantable CRM device requesting the current list of available programs.

Another aspect relates to a method, at an implantable cardiac rhythm management, CRM, device. The method may comprise receiving, from a patient device, a query to switch to one or more available programs, wherein the one or more available programs are stored in a memory of the implantable CRM device.

The method, at the implantable CRM device may comprise sending, to the patient device, a current list of available programs of the implantable CRM device. Optionally, the method may further comprise receiving a request, from the patient device, requesting the current list.

Another aspect of the present invention relates to a method, at a programming device, for programming an implantable cardiac rhythm management, CRM, device and the method may comprise sending programming data to the implantable CRM device and may comprise providing the implantable CRM device with a set of programs.

The sending of the programming data to the implantable CRM device (at the programming device) may further comprise sending the programming data remotely, via a remote network service and/or a relay device.

Another aspect relates to a method which may be performed by a system comprising a patient device as outlined herein and an implantable cardiac rhythm management, CRM, device (as outlined herein). The method may comprise at least one of the method steps at the patient device (as outlined herein) and may comprise at least one of the method steps at the implantable CRM device (as outlined herein). In addition, it may optionally also be possible that the method relates to a method performed by a system that also comprises a programming device as outlined herein and comprises at least one of the method steps at the programming device (as outlined herein).

Another aspect relates to a method which may be performed by a programming device for programming an implantable cardiac rhythm management, CRM, device. The method may include receiving, at the programming device, a user input via a user interface of the programming device to a remote network service. It may further include, causing, by the user interface, the remote network service to provide the implantable CRM device with a set of programs. The causing may be carried out in response to receiving the user input.

It is noted that this aspect may be combined with any of the further method steps outlined herein with reference to a programming device.

The present disclosure further comprises a computer program which may comprise instructions which may cause a computer to implement the method steps described herein, and/or the means as described herein, when the instructions are executed.

The query, as described herein, may be understood, e.g., as a (dedicated) data structure that may be understood by the implantable CRM device and the patient device. Optionally, it may also be understood by the relay device and or the remote network service as described herein. However, the latter may also be transparent for the query. The query may be structured such that the implantable CRM device is able to uniquely determine the program(s) to which it shall switch.

The present disclosure is primarily described with reference to a CRM device. However, it is noted that it may also be applied to other implantable devices.

For example, an aspect of the present disclosure may relate to a patient device for adjusting settings of an implantable device. The patient device may comprise a memory with a list of available programs of the implantable device, and it may comprise a user interface for selecting, by the patient, one or more of the available programs. The patient device may further comprise a transmitter for sending a query to the implantable device to switch to the one or more selected programs. The patient device may be additionally configured with aspects as outlined herein (without necessarily relating to a CRM device).

Particularly, as an example, the patient device may further comprise a receiver for receiving a current list of available programs of the implantable device from the implantable device. The transmitter may further optionally be configured to send a request to the implantable device requesting the current list.

As another example, an aspect of the present disclosure may relate to an implantable device comprising a memory with available programs of the implantable device and a receiver for receiving, from a patient device, a query to switch to one or more of the programs. The implantable device may be additionally configured with aspects as outlined herein (without necessarily relating to a CRM device).

Particularly, as an example, the implantable device may further comprise a transmitter for sending, to the patient device, a current list of available programs of the implantable CRM device. Further, the receiver of the implantable device may optionally be configured for receiving a request, from the patient device, requesting the current list of available programs of the implantable CRM device.

It is noted that the method steps as described herein may include all aspects described herein, even if not expressly described as method steps but rather with reference to an apparatus. Moreover, the devices as outlined herein may include means for implementing all aspects as outlined herein, even if these may rather be described in the context of method steps.

Whether described as device functionalities, method steps, computer program and/or generic means, the functions described herein may be implemented in hardware, software, firmware, and/or combinations thereof. If implemented in software/firmware, the functions may be stored on or transmitted as one or more instructions or code on a computer-readable medium. Computer-readable media includes both computer storage media and communication media including any medium that facilitates transfer of a computer program from one place to another. A storage medium may be any available media that can be accessed by a general purpose or special purpose computer. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor.

It is further noted that the present invention is not limited to the specific feature combinations expressly listed herein, which are only understood as examples. Other features and/or feature combinations may also be possible.

The following figures are provided to support the understanding of the present invention:
- Fig. 1: Illustration of a first possible embodiment of the devices/methods according to the present disclosure.
- Fig. 2: Illustration of a second possible embodiment of the devices/methods according to the present disclosure.
- Fig. 3: Illustration of a third possible embodiment of the devices/methods according to the present disclosure.

The following detailed description and further embodiments described therein are provided to facilitate the understanding of the invention.

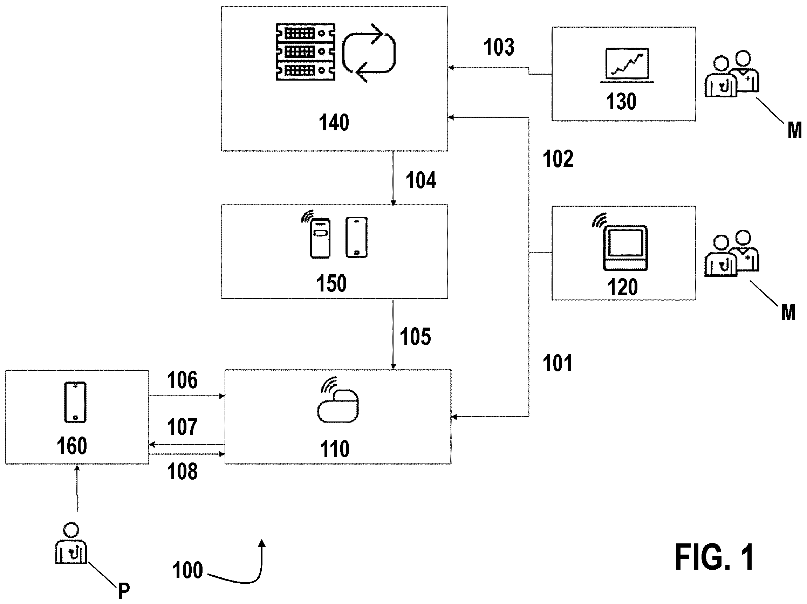

Fig. 1 shows a diagram of a first exemplary embodiment 100 of the aspects described herein and possible steps carried out by possible devices according to the present disclosure.

An implantable CRM device 110 may be provided. It may comprise a memory which has stored thereon a plurality of (sub-)programs. By selecting and running one or more available programs, stored on the implantable CRM device 110, the CRM device 110 may provide various operation parameters or modes corresponding to the selected program.

The memory may be implemented as a transient and/or a non-transient memory. The storage of (available) programs may be organized such that the programs are stored as/in at least one of configuration files (e.g. XML, JSON or any other suitable file format), database entries, in a blockchain, etc.

A programming device 120 may be provided. Programming device 120 may be understood as an, e.g. handheld, device which may for example mainly be used by medical staff M. Programming device 120 may relate to a dedicated handheld device for sending programming data to the implantable CRM, a mobile phone (e.g. a smartphone which may execute a dedicated app), a tablet, a (desktop) computer/terminal, or any other suitable device.

The programming data and programs may be generated and/or modified by means of programming device 120 by at least one member of medical staff M. The medical staff M may relate to doctors, nurses, etc. The medical staff in the given context may however also comprise software developers/programmers who are entitled to develop one or more programs for the implantable medical device, e.g., in cooperation with doctors.

Programming device 120 may comprise a transmitter to transmit 101 programming data and thus one or more programs generated on the programming device 120 (or merely modified or selected for provision to the implantable CRM device 110), to the implantable CRM device 110. Implantable CRM device 110 may then provide, e.g. automatically, a current list of programs to a patient device 160 (as described further below).

The transmitter may be implemented as a near field communication (NFC) interface, a Bluetooth interface, a Wi-Fi interface or any other suitable interface for direct transmission to implantable CRM device 110.

Implantable CRM device 110 may comprise a corresponding receiver and may thus receive 101 the programming data directly (e.g. without any further entities in between the implantable CRM device 110 and the programming device 120).

The one or more programs may instantaneously (neglecting potential transmission latency) be transmitted to the implantable CRM device 110. Alternatively, it may be possible that the implantable CRM device 110 receives the programming data asynchronously (i.e., after certain criteria are met, e.g. a proximity of the implantable CRM device 110, etc.).

As an alternative or additionally, it may be possible that the programming device 120 is in indirect communication with the implantable CRM device 110 and transmits 102 programming data (and/or at least a portion thereof) to a remote network service 140. Hence, the updated programming data may thus also be available on remote network service 140. Remote network service 140 may, however, also forward the data to implantable CRM device 110 (as will be described further below). Programming device 120 may comprise a respective transmitter/interface to allow the required communication. The transmitter may be implemented to allow a network and/or an internet connection. The connection may be implemented as a wired and/or wireless connection to the network and/or the internet. A wired connection may e.g. relate to an Ethernet connection, a fibre connection and/or any other suitable wired connection technique. A wireless connection may e.g. relate to Wi-Fi, 3G, 4G, 5G and/or any other wireless communication technique.

Indirect communication may allow to modify the programs on the CRM device 110 and/or to add programs at any time, e.g. after implantation, without the patient having to visit a doctor.

Also, indirect communication, may for example allow an update of programs not only at a single implantable CRM device 110 at a time. Instead, a plurality of updates may be provided to various devices at the same time. This may be useful in cases in which the medical staff M does not create programs exclusively for an individual patient but for a potential application in a variety of implantable CRM devices carried by various patients.

Additionally or alternatively, it may be possible to provide a second programming device 130. Second programming device 130 may only communicate 103 with implantable CRM device 110 via remote network service 140. It may be implemented similarly as programming device 120. However, it is also possible to implement it as a mainly stationary device, e.g. as a desktop computer, e.g. in a hospital environment. Also, it is possible to implement it as a mobile, but not necessarily handheld device, e.g. a laptop computer or tablet, e.g., such as to facilitate convenient programming, e.g. via a larger display, keyboard etc., compared to a possibly more compact handheld programming device 120.

The second programming device 130 may also possess a respective transmitter/interface (as described above) such that a communication with the remote network service 140 may be established as a network connection, over the internet and/or any other suitable communication technique. The transmission of the one or more programs may be performed instantaneously or asynchronously.

One or more programs may be transmitted 103 as programming data from programming device 130 to the remote network service 140.

Alternatively, the programming device 130 may be a device that may possibly be simpler, e.g. a tablet (or any other mobile, handheld, and/or portable device, or also a simpler stationary device), which (only) provides a user interface to the remote network service 140. Using this user interface, programs can be imported into the remote network service 140 from remote sources or from a source within the remote network service 140 itself, and then sent from there to the implantable CRM device 110. The programs may be generated or, if available, selected within the remote network service. For example, the user interface may be capable of displaying a user interface which, e.g., is provided by the remote network service 140 as, e.g., a webservice (e.g., a website, etc.).

The remote network service 140 may be implemented as described above and may possess one or more transmitters and receivers with interfaces as described herein such that an internet connection to the first programming device 120 and/or the second programming device 130 may be facilitated.

Also, as outlined herein, the remote network service 140 may store programs received from programming devices 120, 130, and programming devices 120, 130 may retrieve programs from remote network service for review and selection and/or modification.

Remote network service 140 may be in communication with relay device 150.

Relay device 150 may be understood as a middleware device which may allow communication between two devices which do not intrinsically possess the ability to communicate with each other directly such as e.g. the implantable device 110 and the remote network service 140. In such a scenario, the relay device 150 may provide an interface for establishing a connection to both the remote network service 140 (e.g. by means of an internet and/or network connection) and to the implantable CRM device 110 (e.g. by means of a near field communication or any other suitable communication technique as outlined above). Relay device 150 may for example provide direct communication with the implantable CRM device 110, similarly as programming device 120. Additionally, it may provide a connection to remote network service 140, similarly as programming device 120.

Thus, relay device 150 may send 105 programming data received from remote network service 140 to implantable CRM device 110.

It may be possible that programming data 103 (and/or programming data 102 and/or programming data 101) is equipped with a patient specific ID. In case programming data 103 (and/or programming data 102) is provided with a patient specific ID, remote network service 140 may assign the received programming data 103 (and/or programming data 102) to a particular patient based at least in part on the patient specific ID. It may selectively forward it to the corresponding relay device 150 which may then transmit it to implantable CRM device 110. CRM device 110 may then automatically provide patient device 160 with a current list of available programs (e.g. as described herein and particularly further below).

It may be possible that the remote network service 140 may automatically inform (e.g. by means of a push notification) the implantable CRM device 110 if a new program has been received at the remote network service 140. The remote network service 140 may establish a communication link 104 with relay device 150. The implantable CRM device 110 may then receive a corresponding transmission from relay device 150. This may allow that patient device 160 (as it will further be described below) and the implantable CRM device 110 are synchronized with respect to the available programs and it may ensure that the patient P is always informed about the latest and potentially best suitable programs for the individual therapy goal of patient P.

Programming of the implantable CRM device 110 may be possible prior to or at the implantation process or may be performed at least in part after the implantation process. It may be required that the patient P is hospitalized during the programming procedure, e.g. for the transmission of programming data 101 to the implantable CRM device 110 which may require a proximity of the patient P (with implantable CRM device 110) to the first programming device 120. Alternatively or additionally, it may also be possible that a remote programming is performed wherein the patient P is not required to be hospitalized (e.g. if the transmission of programs occurs by means of the remote network service 140). Moreover, it may also be possible that the patient P is hospitalized during an initial programming by means of the programming device 120 and may receive further programming data 103 by means of remote programming (e.g. programming device 130) without being hospitalized (or vice versa).

Further, according to Fig. 1, patient device 160 may be in direct communication (i.e. no further entities are in between the patient device 160 and the implantable CRM device 110) with the implantable CRM device 110.

Patient device 160 in the context of the present application may be understood as a device which is suitable for being carried by the patient (and/or a relative and/or a caretaker of the patient). Such a device may e.g. be a mobile phone (e.g. a smartphone which may execute a dedicated app), a tablet, a dedicated handheld device (i.e. a device which has exclusively been developed to interact with the implantable CRM device), a wearable (e.g. a smartwatch or any other bracelet-like device which is capable of providing the required functionality as mentioned above), a smart home device, and/or any suitable mobile and/or handheld device.

Patient device 160 may comprise a user interface. A user interface encompasses any means that allows the patient to select, via the user interface, a program, e.g. one or more (physical) buttons.

Patient device 160 may comprise a graphical user interface (GUI). The GUI may e.g. comprise a display for displaying, e.g., therapy related information and/or a list of programs, to the patient on the patient device 160 and/or to receive an input from the patient. An input may be received by means of one or more physical buttons (e.g., surrounding the display or which are arranged on the patient device in another suitable manner), by means of a touch sensitive screen (e.g. being operable by a finger and/or a stylus), a dial wheel and/or any combination. The user interface may further be capable of informing the patient of the currently selected program, the period of time the current program is active, potential recommendations whether another available program would be better suited for the current health state of the patient, current vital parameters (e.g. heart rate, blood-oxygen-concentration, body temperature, etc.), etc.

The user interface may additionally or alternatively comprise an acoustic interface such that patient device 160 may be operated by acoustic commands. The patient device may also, e.g., "read" (as an acoustic output) any information to be provided to the patient and may thus provide an acoustic output to e.g. the patient. This may facilitate the interaction of patients with the implantable CRM device with restricted ability to see.

Patient device 160 may be in communication with implantable CRM device 110. The communication with implantable CRM device 110 may require a pairing of patient device 160 with implantable CRM device 110. The pairing may ensure that only authorized devices may communicate with each other.

Patient device 160 may transmit 106 a request to the implantable CRM device 110 requesting a current list of available programs of the implantable CRM device 110. In response to transmitting 106 the request (sent to the implantable CRM device 110), the patient device 160 may receive 107 a list of available programs. In other examples, no request 106 may be needed to receive 107 the list of available programs.

After receiving 107 the list of available programs at patient device 160, patient device 160 may indicate the received update by means of an acoustic notification (e.g. a sound, an alarm, etc.) or visual notification (e.g. a blinking flash, a blinking LED, a message on an (e.g. touch sensitive) screen, etc.) such that the patient P is informed that an update has safely been received at the patient device 160.

The data associated with the receiving 107 of the list of available programs may be stored (as a list) on a memory (the memory may be implemented similar to the memory of the implantable CRM device 110 as described above) of the patient device 160.

Based at least in part on receiving 107 the list of available programs, patient P may select one or more of the available programs by means of the patient device 160 and the input/output means as described above. For example, it may be possible that a GUI may be implemented to display the received data based on receiving 107 the list of available programs on a touch sensitive display. Patient P may select one or more programs by means of a touch input (e.g. by a finger).

The selection of one or more programs at the patient device 160 may be sent as a query 108, from the patient device 160, to the implantable CRM device 110. The sending 108 of the query to the implantable CRM device 110, may preferably be encoded such that redundancy checks may be performed upon receiving the query at the implantable CRM device. It may also be possible that the implantable CRM device returns an acknowledgement (ACK) notification upon a safe and decodable reception of the query (or a non-acknowledgment (NACK) notification if a failure has been determined). Moreover, encoding of the query also ensures that no unauthorized entity (e.g. an unauthorized person) may alter CRM device 160. Similarly, also transmissions 106 and/or 107 may be encoded.

Each of the devices (e.g., but not limited, to patient device 160 and implantable CRM device 110 as mentioned above) may be assigned with an identification number (ID) which is also saved in a centralized location (e.g. the remote network service 140 or any other suitable location and/or database). The centralized location may further store information directed to the aspect of which devices (encoded by means of their ID) are allowed to communicate with each other.

As an example, prior to an establishing of a communication between the patient device 160 and the implantable CRM device 110, an allowance request may be transmitted from the implantable CRM device 110 to the centralized location. This allowance request may comprise the IDs of the two devices which are pending to establish a connection. In response to the allowance request, the implantable CRM device 110 may receive an allowance or a decline for establishing the communication with the patient device 160. The allowance request may only be sent once (e.g. when an initial communication between two devices is established) or may be sent each time a communication is established.

The implantable CRM device 110 may be capable of identifying the desired one or more programs to be executed from the query. The implantable CRM device 110 may then execute and/or switch to the respective one or more programs as stored on the implantable CRM device 110.

The switching to one or more available programs, at the implantable CRM device 110, may comprise saving the current settings of the currently active one or more programs (and/or the main program) prior to switching to the queried one or more available programs. This may allow that the implantable CRM device 110 may safely return to the original one or more programs (e.g., as outlined above).

It may also be possible, that the implantable CRM device 110 possesses an internal surveillance procedure which may track certain vital parameters of the patient for a pre-defined amount of time upon switching to one or more available programs to ensure that the "new" active programs do not cause an undesired deterioration of the health state of the patient. If a deterioration is determined, the implantable CRM device 110 may return to the original one or more programs (as saved beforehand). This may increase the overall patient safety when interacting with the implantable CRM device 110.

It is noted that the possibility to alter programs on implantable CRM device 110 may selectively be enabled or disabled for each individual implantable CRM device 110, e.g. permanently or temporally. For example, this may be done by medical staff in connection with the implantation process, wherein it may be decided, for each patient, whether the functionality shall be enabled. Also, enabling or disabling may be implemented remotely, e.g. via a corresponding message transmitted to remote network service 140 and/or directly to implantable CRM device 110, e.g. by programming device 120 and/or 130.

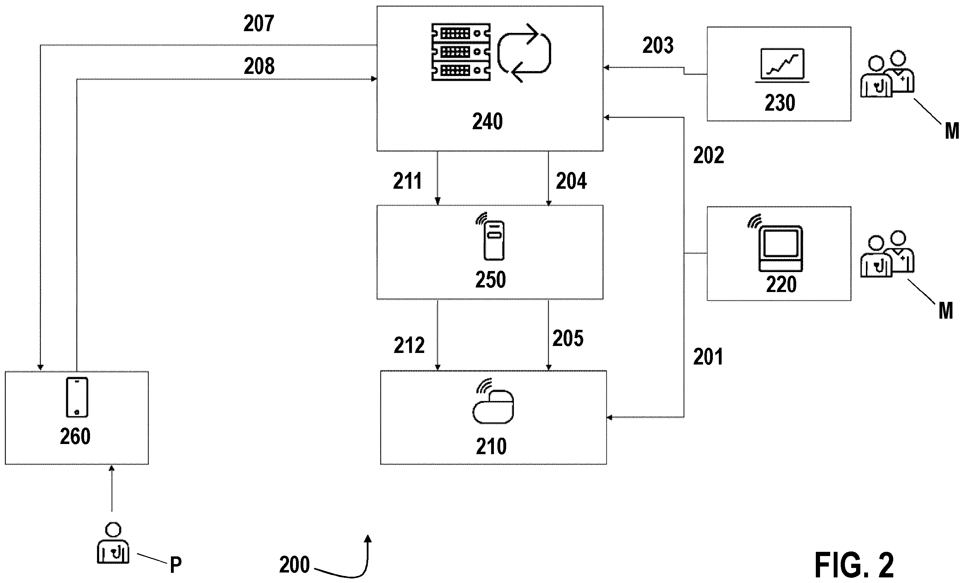

Fig. 2 shows a diagram of a second exemplary embodiment 200 of devices according to the present disclosure and corresponding method steps. It is noted that the second embodiment, as it will be further described below, may be understood as an alternative or an addition to the first embodiment as described above.

Devices 210, 220, 230, 240, and 250 may be understood as being similar or identical to the respective devices 110, 120, 130, 140, and 150 as discussed with reference to Fig. 1. The steps 201, 202, 203, 204 and 205 may be similar or identical to steps 101, 102, 10, 104 and 105 described with reference to Fig. 1. Hence, those will not be discussed in further detail below.

However, the second embodiment 200 may be understood as providing an additional and/or an alternative application scenario how patient P may interact (by means of a patient device 260) with the implantable CRM device 210. All aspects of the first embodiment (as described above) may additionally or alternatively be applicable to the second embodiment as described herein below.

Reference numeral 260 designates the patient device 260 that may also generally be similar to patient device 160 described above with reference to Fig. 1. In particular, all aspects described above with reference to patient device 160, may also be implemented in patient device 260. However, additionally or alternatively to receiving 107 a current list of programs from implantable CRM device 110 directly, patient device 260 may receive 207 the list from the remote network service 240.

For example, implantable CRM device 210 may be configured to provide the current list to remote network service 240 regularly and/or each time it is updated at implantable CRM device 210. Additionally or alternatively, remote network service 240 may query implantable CRM device 210 regularly and/or each time it receives programming data 202, 203, such as to provide the current list.

In some examples, as outlined above, remote network service 240, whenever implantable CRM device 210 is provided 201 with programming data directly, the programming data may also be sent 302 to remote network service. In such examples, remote network service 240 may always be aware of the current list, without having to inquire with implantable CRM device 210.

Remote network service 240 may be configured to send the current list to patient device 260 regularly and/or whenever remote network service 240 receives a corresponding update (e.g. the list stored in the remote network service 240 changes). Regularly may be understood as e.g. based on a pre-defined schedule (e.g. certain dates) and/or periodically (e.g. once per hour, once per day, once per week, once per month, once per year, etc.). Before sending the current list to patient device 260, remote network service 240 may optionally verify that the list is indeed current, e.g. by sending a corresponding message to that implantable device 210 and receiving a corresponding response.

Additionally or alternatively, it may also be possible that the medical staff M initiates transmission 207 of the current list, e.g., via a request sent by programming device 202 and/or 203.

Similarly as described for embodiment 100, it may also be possible that the patient initiates an update of the list of available programs for the implantable CRM device 210 with a corresponding request (not shown in Fig. 2). Patient P may initiate the receiving 207 of the current list of available programs by means of patient device 260, e.g., by sending a request to remote network service 240 requesting a list of currently available (and potentially updated) programs of the implantable CRM device 210.

Patient P may select, at patient device 260, one or more desired programs from a list of potentially available programs based at least in part based on the receiving 207 of the list of available programs.

Based on the selection, patient device 260 may send a query 208 to implantable CRM device 210 by means of the remote network service 240 (e.g. over an ethernet and/or internet connection as described above). Thus, in this second possible embodiment 200, the patient may indirectly interact with the implantable CRM device 210.

The remote network service 240 may forward the query 211 to the implantable CRM device 210 by means of relay device 250, acting as a middleware device.

Relay device 250 may then forward the query 212 to the implantable CRM device 210.

In response to receiving the request 212 from the patient device 260, implantable CRM device 210 may activate the queried one or more programs (e.g., as outlined above).

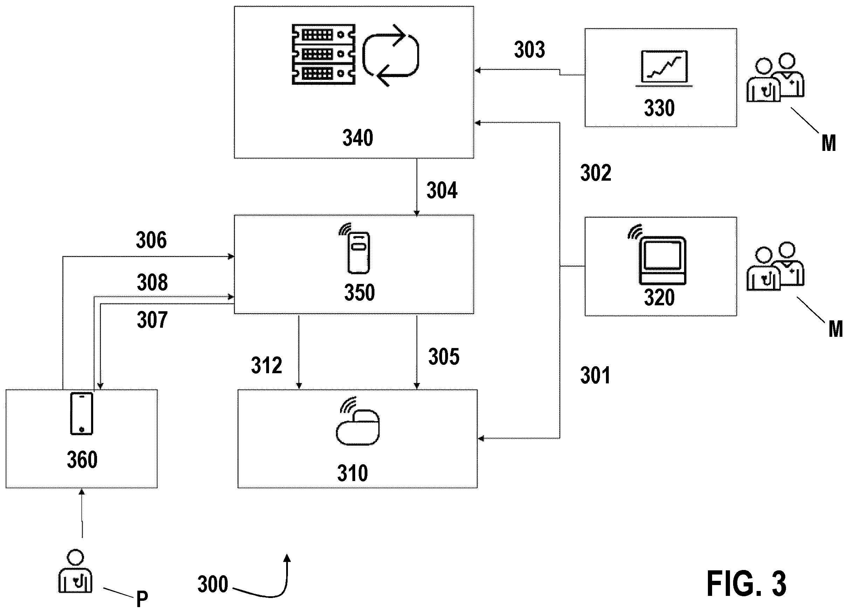

Fig. 3 shows a diagram of a third exemplary embodiment 300 of devices and method steps according to the present disclosure. It is noted that the third embodiment, as it will be further described below, may be understood as an alternative or an addition to the first and second embodiment as described above.

Devices 310, 320, 330, 340, and 350 may be similar or identical to the respective devices 110, 120, 130, 140, and 150 as discussed with reference to Fig. 1 or to the respective devices 210, 220, 230, 240, and 250 as discussed with reference to Fig. 2. The steps 301, 302, 303, 304 and 305 may be similar or identical to steps 101, 102, 103, 104 and 105 described with reference to Fig. 1 or to steps 201, 202, 203, 204 and 205 described with reference to Fig. 2.

This third embodiment 300 may be understood as providing an additional and/or an alternative application scenario with respect to the first and/or the second embodiment as described above how the patient P may interact (by means of a patient device 360) with the implantable CRM device 310. All aspects of the first embodiment (as described above) and/or the second embodiment (as described above) may additionally or alternatively be applicable to the third embodiment as described herein below.

Reference numeral 360 designates patient device 360 that may also generally be similar to patient device 160 and/or 260 described above with reference to Figs. 1 and 2. In particular, all aspects described above with reference to patient device 160 and/or 260 may also be implemented in patient device 360. However, additionally or alternatively to receiving 107 a current list of programs from implantable CRM device 110 directly or via remote network service 240, patient device 360 may receive 307 the list from the relay device 350.

Similarly as outlined above (e.g. for the first embodiment 100), patient device 360 may transmit 306 a request requesting a list of potentially available programs, from implantable CRM device 310. In this third embodiment 300, the request is transmitted from patient device 360 to relay device 350 wherein, patient device 360 may communicate indirectly with the implantable CRM device 310 by means of relay device 350.

Relay device 350 may receive 306 the request from patient device 360 and may forward 312 the request to implantable CRM device 310.

Based on the received 312 request, implantable CRM device 310 may return a current list of available programs to relay device 350.

Relay device 350 may in turn forward the list of available programs 307 to patient device 360. In other examples, relay device 350 may forward 307 the current list to patient device 360 without the need for a request 306. This may be done similarly as outlined above with respect to Fig. 1 or Fig. 2. For example, relay device 350 may be provided with the current list by implantable CRM device 310 and/or remote network service 340, e.g. regularly and/or whenever the current list changes.

Similarly as outlined above, based on the received current list of available programs, patient P may select one or more of the available programs, at patient device 360.

Patient device 360 may transmit 308 the request to relay device 350, which in turn may forward the request to implantable CRM device 310. Request 308 may comprise an indication for implantable CRM device 310 to switch to the one or more programs selected by patient P.

In response to receiving the query, implantable CRM device 310 may execute/switch to the one or more programs indicated in the query as described above.

## Claims

1. A patient device (160; 260; 360) for adjusting settings of an implantable cardiac rhythm management, CRM, device (110; 210; 310) comprising:
a memory with a list of available programs of the implantable CRM device (110; 210; 310);
a user interface for selecting, by the patient (P), one or more of the available programs;
a transmitter for sending (108; 208; 308) a query to the implantable CRM device (110; 210; 310;) to switch to the one or more selected programs.

2. The patient device (160; 260; 360) of claim 1,
wherein the transmitter is configured to send (106; 306) a request to the implantable CRM device (110; 210; 310) requesting a current list of available programs of the implantable CRM device (110; 210; 310);
wherein the patient device further comprises a receiver for receiving (107; 207; 307) the current list from the implantable CRM device (110; 210; 310).

3. An implantable cardiac rhythm management, CRM, device (110; 210; 310), comprising:
a memory with available programs of the implantable CRM device (110; 210; 310);
a receiver for receiving (108; 208; 308), from a patient device (160; 260; 360), a query to switch to one or more of the programs.

4. A programming device (130, 230, 330) for programming an implantable cardiac rhythm management, CRM, device (110; 210; 310), comprising: a user interface to a remote network service (140, 240, 340), wherein the user interface is configured to cause the remote network service (140, 240, 340) to provide the implantable CRM device (110; 210; 310) with a set of programs.

5. A programming device (120, 130; 220, 230; 320, 330) for programming an implantable cardiac rhythm management, CRM, device (110; 210; 310), comprising:
a transmitter for sending (101, 102, 103; 201, 202, 203; 301, 302, 303) programming data to the implantable CRM device (110; 210; 310); wherein the programming device (120, 130; 220, 230; 320, 330) is adapted to provide the implantable CRM device (110; 210; 310) with a set of programs.

6. The programming device (120, 130; 220, 230; 320, 330) of claim 5, wherein the transmitter is adapted to send (102, 103; 202, 203; 302, 303) the programming data to the implantable CRM device (110; 210; 310) remotely, via a remote network service (140; 240; 340) and/or a relay device (150; 250; 350).

7. A system for adjusting settings of a cardiac rhythm management, CRM, device (110; 210; 310), comprising the patient device (160; 260; 360) of claim 1 or 2 and at least one of:
the implantable cardiac rhythm management, CRM, device (110; 210; 310) according to claim 3;
the programming device (120, 130; 220, 230; 320, 330) for programming an implantable cardiac rhythm management, CRM, device (110; 210; 310) according to claim 4, 5 or 6.

8. A method, at a patient device (160; 260; 360), for adjusting settings of an implantable cardiac rhythm management, CRM, device (110; 210; 310), comprising:
selecting, based on patient (P) input provided via a user interface of the patient device (160; 260; 360), one or more programs from a list of available programs for the implantable CRM device (110; 210; 310) stored in a memory of the patient device (160; 260; 360);
sending (108; 208; 308) a query to the implantable CRM device (110; 210; 310) to switch to the one or more selected programs.

9. The method of claim 8, further comprising:
sending (106; 306) a request to the implantable CRM device (110; 210; 310) to request a current list of available programs of the implantable CRM device (110; 210; 310);
receiving (107; 207; 307) the current list from the implantable CRM device (110; 210; 310).

10. A method, at an implantable cardiac rhythm management, CRM, device (110; 210; 310), comprising: receiving (108; 208; 308), from a patient device (160; 260; 360), a query to switch to one or more available programs, wherein the one or more available programs are stored in a memory of the implantable CRM device (110; 210; 310).

11. The method of claim 10, further comprising:
receiving (206; 306) a request, from the patient device (160; 260; 360), requesting a current list of available programs of the implantable CRM device (110; 210; 310);
sending (107; 207; 307), to the patient device (160; 260; 360), the current list.

12. A method, at a programming device (120, 130; 220, 230; 320, 330), for programming an implantable cardiac rhythm management, CRM, device (110; 210; 310), comprising:
sending (101, 102, 103; 201, 202, 203; 301, 302, 303) programming data to the implantable CRM device (110; 210; 310);
such that the implantable CRM device (110; 210; 310) is provided with a set of programs.

13. The method of claim 12, wherein sending (102, 103; 202, 203; 302, 303) the programming data to the implantable CRM device (110; 210; 310) further comprises sending (102, 103; 202, 203; 302, 303) the programming data remotely, via a remote network service (140; 240; 340) and/or a relay device (150; 250; 350).

14. A method performed by a system comprising a patient device (160; 260; 360) and an implantable cardiac rhythm management, CRM, device (110; 210; 310) comprising the steps of claims 8 or 9 and the steps of claims 10 or 11.

15. A computer program, comprising computer executable code, which when executed by a computer, performs a method according to one of claims 8 to 14.
